# EUROPEAN PATENT APPLICATION

(11) **EP 4 585 172 A1**
(43) Date of publication of application: **16.07.2025**
(21) Application number: 24809539.0
(22) Date of filing: 15.03.2024
(51) Int. Cl.: A61B 17/435, A61B 17/43, A61M 3/02, A61M 5/142, A61M 5/14, A61M 1/00

(54) **EASY-TO-OPERATE AUTOMATIC FLUSHING STRUCTURE AND OOCYTE ACQUISITION DEVICE**

(30) Priority: 30.11.2023 CN 202311619278
(71) Applicant: Tongye Technologies Development Co., Ltd., Binhai New Area, Tianjin 300384 (CN)
(72) Inventor: YIN, Hua, Tianjin Shi 300384 (CN); ZHANG, Ying, Tianjin Shi 300384 (CN); ZHAI, Guanlin, Tianjin Shi 300384 (CN); MA, Jin, Tianjin Shi 300384 (CN); LI, Tianming, Tianjin Shi 300384 (CN)
(74) Representative: Bryn Aarflot AS
(86) International application number: PCT/CN2024/081961
(87) International publication number: WO 2025/112234

(57) **Abstract**

Provided is a conveniently operable automatic flushing structure, including a mounting housing (2), a syringe driving mechanism, and a syringe clamping mechanism. The mounting housing (2) is a of structure with clamping groove disposed on a side thereof, the syringe clamping mechanism is arranged outside the clamping groove, and the syringe driving mechanism is arranged inside the mounting housing (2). An oocyte collection instrument is further provided, including a collection instrument body, the automatic flushing structure is arranged on a side of the collection instrument body.

## Description

### TECHNICAL FIELD

The present disclosure belongs to the field of medical devices, relates to a device for reproductive surgery, and in particular to a conveniently operable automatic flushing structure, and an oocyte collection instrument.

### BACKGROUND

Oocyte collection instrument is special equipment developed for extracting oocytes in assisted reproductive surgery. Currently, most of the oocyte collection instruments in the market only have the function of oocyte retrieval, and the flushing is performed by injecting liquid manually with the needle tubing, which is inconvenient to operate.

Chinese Patent Application Publication No. 214966446U discloses an ovum negative pressure suction device for oocyte retrieval operation. A flushing mechanism on the negative pressure suction device includes a peristaltic pump driven by a stepping driving all-in-one machine and a flushing liquid test tube. The peristaltic pump is installed on an upper housing on the side surface of a casing, and a liquid inlet end of the peristaltic pump is communicated with the flushing liquid test tube through a pipeline, and a liquid outlet end of the peristaltic pump is communicated with a double-lumen oocyte retrieval needle through a pipeline. Although the function of automatic flushing is achieved, it is necessary to connect sterile tubes connected to the flushing liquid test tube and the double-lumen oocyte retrieval needle respectively to the peristaltic pump in clinical practice, causing inconvenience in preparation before oocyte retrieval.

In view of this, a conveniently operable automatic flushing structure and an oocyte collection instrument are provided, which can further facilitate the oocyte retrieval operation and improve the efficiency of oocyte retrieval.

### SUMMARY

An objective of the present disclosure is to provide a conveniently operable automatic flushing structure and an oocyte collection instrument, so as to overcome the problems described in the background.

The technical problem is solved by the following technical solution in the present disclosure:
A conveniently operable automatic flushing structure includes a mounting housing, a syringe driving mechanism, and a syringe clamping mechanism. The mounting housing is a of a structure with clamping groove disposed on a side thereof, the syringe clamping mechanism is arranged outside the clamping groove, and the syringe driving mechanism is arranged inside the mounting housing.

Moreover, the syringe driving mechanism includes a guide rail frame, a motor, a push handle driving lead screw, a push handle of a syringe, guide rods, a push rod, and a meshing assembly. The push handle driving lead screw driven by the motor is installed inside the guide rail frame, a triangular slider is sleeved on the push handle driving lead screw, and two end corners of the triangular slider are sleeved on the guide rods installed inside the guide rail frame in an up and down direction, and an other end corer of the triangular slider is rotatably connected with the push rod. The push handle of the syringe is connected to an end of the push rod, and the meshing assembly is arranged on a side of the triangular slider. The push rod is configured to control the meshing assembly to be meshed with and separated from the push handle driving lead screw through a bump arranged on the push rod, and a limit optocoupler is installed on one end, away from the motor, of an inner wall of the guide rail frame.

Moreover, the meshing assembly includes a meshing block, a compression spring, and a pressing plate. The meshing block includes a hinged portion, a meshing portion, and a crimping portion. The meshing portion and the crimping portion are arranged on two ends of the hinged portion, respectively. The hinged portion is hinged on the side of the triangular slider. A lower end face of the meshing portion is in fit with threads on the push handle driving lead screw, and an upper end face of the meshing portion is connected to the pressing plate through the compression spring. The pressing plate is fixedly installed on the triangular slider; and the crimping portion is placed below the push rod.

Moreover, the syringe clamping mechanism includes a clamping plate, a spring, a baffle plate, and a screw. The clamping plate includes a clamping plate body, and a telescopic shaft arranged below an inner side of the clamping plate body. A telescopic hole is formed below a middle of the mounting housing. The telescopic shaft of the clamping plate is inserted into the telescopic hole through the spring. The baffle plate abutting against the spring is installed on an end of the telescopic shaft through the screw.

An oocyte collection instrument is further provided, including a collection instrument body, where an automatic flushing structure is arranged on a side of the collection instrument body.

Moreover, a negative pressure energy storage tank in the collection instrument body is a stepless adjustable energy accumulation tank. The stepless adjustable energy accumulation tank includes an energy accumulation tank body, an air nozzle, a piston, a piston driving lead screw, a stepping motor, a bracket, an optocoupler, an optocoupler base, and an optocoupler baffle. The air nozzle is provided on a front end of the energy accumulation tank body, and the piston is arranged inside the energy accumulation tank body. The piston is driven to expand and retract by the stepping motor arranged at a rear of the energy accumulation tank body through the piston driving lead screw, and the optocoupler baffle is connected to a rear end of the piston driving lead screw. A lower portion of the optocoupler baffle is provided with the optocoupler base, and the optocoupler base is connected to the bracket by a screw. The bracket is installed inside the collection instrument body by a screw, and the optocoupler is arranged on a rear end of the optocoupler base.

Moreover, a central microprocessor controller is arranged inside the collection instrument body, a suction pedal, a negative pressure value setting keyboard, a flushing pedal and a pressure sensor are connected to an input end of the central microprocessor controller. A liquid crystal display on the collection instrument body is bidirectionally connected to the central microprocessor controller. The automatic flushing structure, a negative pressure pump, an automatic pressure-regulating controller, a venting valve, the stepless adjustable energy accumulation tank, and an on-off valve are connected to an output end of the central microprocessor controller.

The present disclosure has the advantages and beneficial effects as follows:

The conveniently operable automatic flushing structure and the oocyte collection instrument are used in conjunction with a disposable oocyte retrieval needle during flushing operation, and the oocyte collection instrument provides a negative pressure source with constant pressure and stable airflow for the disposable oocyte retrieval needle. The collection instrument employs a stepless adjustable energy accumulation tank, and the capacity of the energy accumulation tank can be adjusted in stepless as needed. It is easier to establish a target negative pressure when the capacity is small, and an output negative pressure is more stable when the capacity is large. The oocyte collection instrument employs the automatic flushing structure to inject liquid during surgical flushing, so that the liquid accuracy is high, the flow velocity is stable, the manpower is saved, the use of pump tube consumables which are indispensable for liquid injection of the peristaltic pump is reduced, and the problem of inconvenient disinfection and sterilization of pump tubes is avoided at the same time.

### BRIEF DESCRIPTION OF THE DRAWINGS

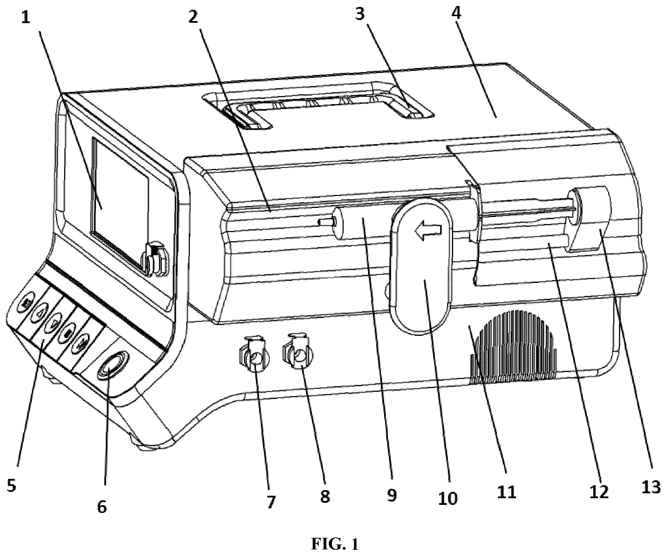

FIG. 1 is a structural diagram of an oocyte collection instrument according to the present disclosure;

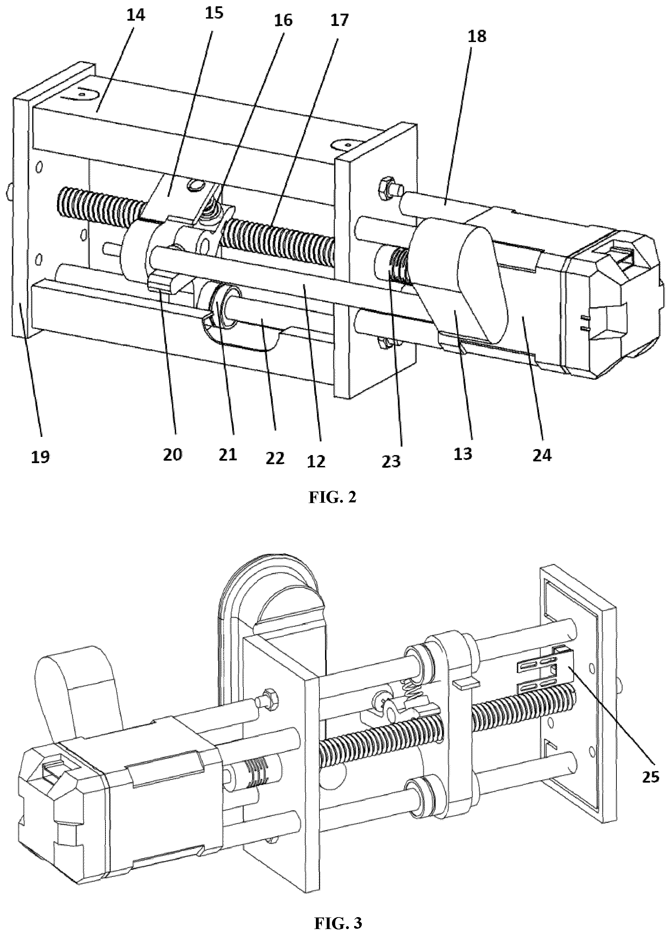

FIG. 2 is a structural diagram of an automatic flushing structure according to the present disclosure;
FIG. 3 is a structural diagram of an automatic flushing structure according to the present disclosure viewed in another angle;

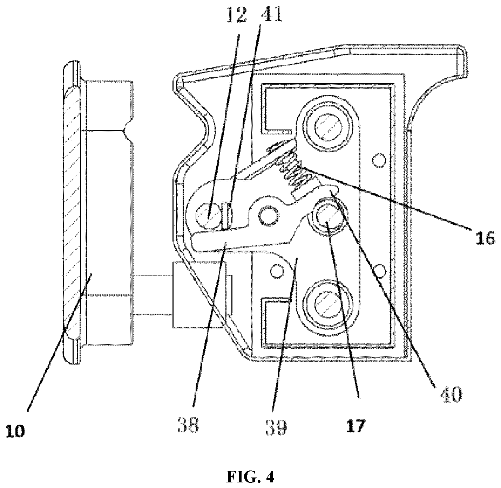

FIG. 4 is a structural diagram of a meshing assembly of the automatic flushing structure according to the present disclosure;

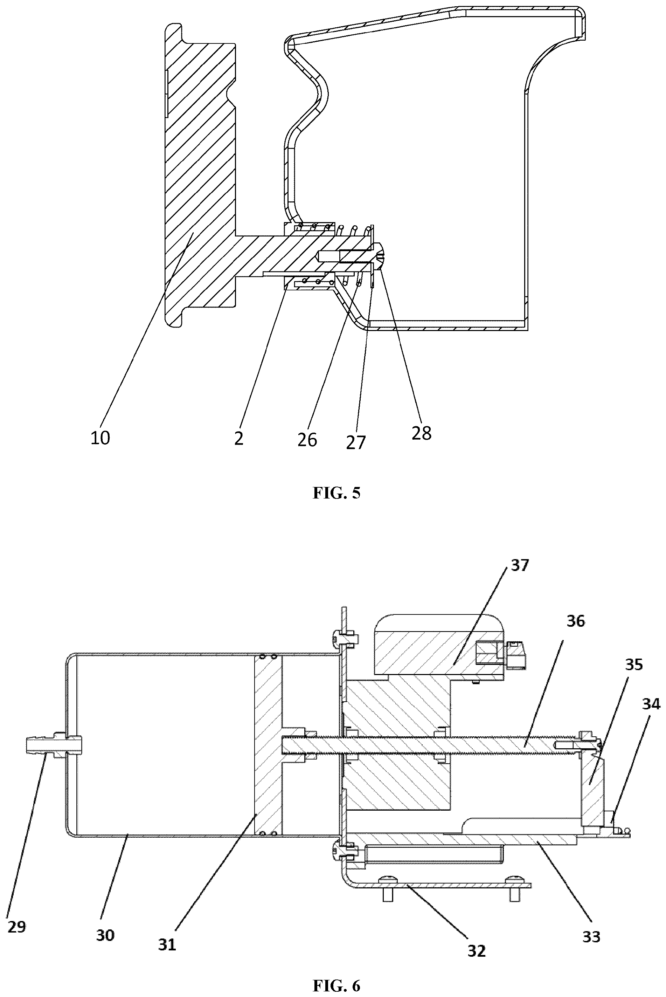

FIG. 5 is a structural diagram of the assembly of a syringe clamping mechanism of the automatic flushing structure according to the present disclosure;
FIG. 6 is a structural diagram of a stepless adjustable energy accumulation tank according to the present disclosure;

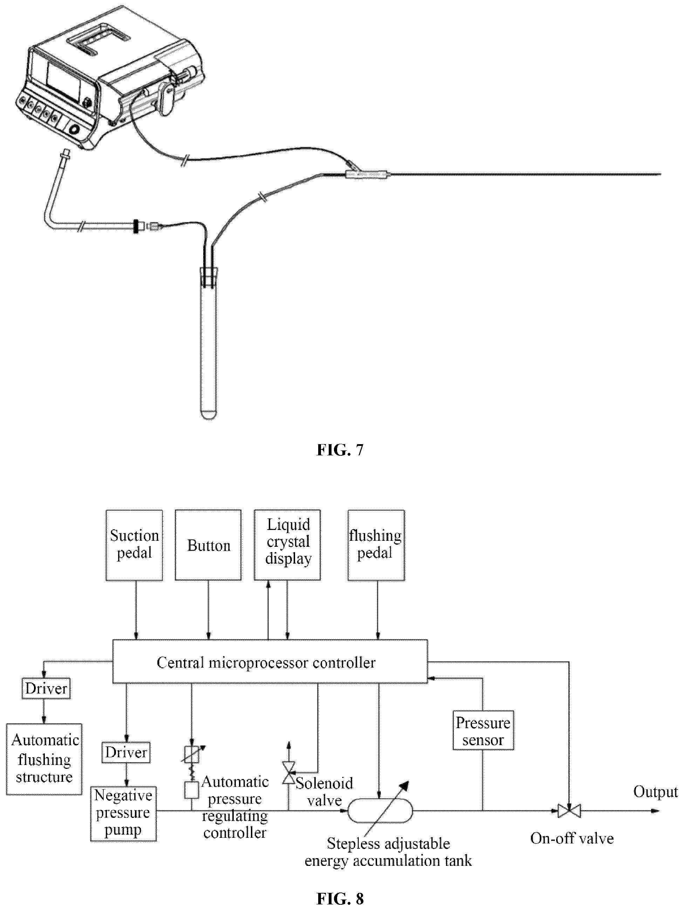

FIG. 7 is a connection diagram of the present disclosure in operation;
FIG. 8 is an operating principle diagram of the present disclosure; and

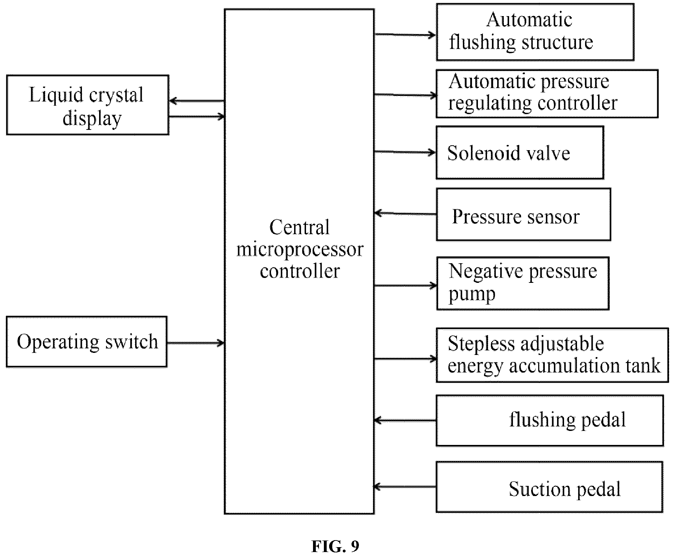

FIG. 9 is a principle block diagram of the present disclosure.

### Reference numerals:

1-liquid crystal display; 2-mounting housing; 3-lifting handle; 4-upper cover; 5-button; 6-operating switch; 7-pedal flushing port; 8-pedal suction port; 9-syringe; 10-clamping plate; 11-bottom cover; 12-push rod; 13-push handle of syringe; 14-guide rail frame; 15-pressing plate; 16-compression spring; 17-push handle driving lead screw; 18-pillar; 19-blocking head of guide rail frame; 20-meshing block; 21-linear bearing; 22-guide rod; 23-coupling; 24-motor; 25-limit optocoupler; 26-spring; 27-baffle plate; 28-screw; 29-air nozzle; 3-energy accumulation tank body; 31-piston; 32-bracket; 33-optocoupler base; 34-optocoupler; 35-optocoupler baffle; 36-piston driving lead screw; 37-stepping motor; 38-crimping portion; 39-triangular slider; 40-meshing portion; 41-bump.

### DETAILED DESCRIPTION OF THE EMBODIMENTS

The following clearly and completely describes the technical solutions in the embodiments of the present disclosure with reference to the accompanying drawings in the embodiments of the present disclosure. All other embodiments obtained by a person of ordinary skill in the art based on the embodiments of the present disclosure without creative efforts shall fall within the protection scope of the present disclosure.

It should be noted that unless otherwise stated, technical terms or scientific terms used in the present disclosure shall have their ordinary meanings as understood by those of ordinary skill in the art.

An oocyte collection instrument includes a collection instrument body. The collection instrument body includes a collection instrument housing, a liquid crystal display 1, buttons 5, an operating switch 6, a pedal flushing port 7, a pedal suction port 8, and a lifting handle 3. The collection instrument housing is formed by buckling an upper cover 4 and a bottom cover 11 in an up and down direction. A front end of the upper cover is provided with the liquid crystal display; an upper end of the upper cover is provided with the lifting handle; a front end of the bottom cover is provided with the buttons and a side end of the bottom cover is provided with the pedal flushing port and the pedal suction port, and the inventive feature of the oocyte collection instrument is that a side of the upper cover is provided with an automatic flushing structure.

The automatic flushing structure includes a mounting housing 2, a syringe driving mechanism, and a syringe clamping mechanism. The mounting housing is installed on a side of the upper cover, and the mounting housing is of a structure with clamping groove disposed the outer side thereof. The syringe clamping mechanism is arranged outside the clamping groove, and the stable clamping of the syringe 9 is achieved through the matching of the syringe clamping mechanism and the clamping groove. The syringe driving mechanism is arranged inside the mounting housing, and the syringe driving mechanism is configured to drive an injection push rod of the syringe, thus achieving the injection of the liquid.

The syringe driving mechanism includes a guide rail frame14, a motor 24, a push handle driving lead screw 17, a push handle 13 of a syringe, guide rods 22, a push rod 12, and a meshing assembly. The push handle driving lead screw driven by the motor is installed inside the guide rail frame, the motor is connected to the push handle driving lead screw through a coupling 23, the motor is installed on the guide rail frame through pillars 18, and a triangular slider 39 is sleeved on the push handle driving lead screw. Each of three end corners of the triangular slider is provided with a via hole, in which two end corners of the triangular slider are provided with linear bearings 21 sleeving the guide rods 22 installed inside the guide rail frame in an up and down direction, and the push rod is rotatably connected to the other end corner of the triangular slider. The push handle of the syringe is connected to an end of the push rod. A side of the triangular slider is provided with the meshing assembly, and the push rod is configured to control the meshing assembly to be meshed with and separated from the push handle driving lead screw through a bump 41 arranged on the push rod. A limit optocoupler 25 is installed on an inner wall of a blocking head 19 of the guide rail frame arranged at one end, away from the motor, of the guide rail frame.

The meshing assembly includes a meshing block 20, a compression spring 16, and a pressing plate 15. The meshing block 20 includes a hinged portion, a meshing portion 40, and a crimping portion 38. The meshing portion and the crimping portion are arranged on two ends of the hinged portion, respectively. The hinged portion is hinged on the side of the triangular slider. A lower end face of the meshing portion is in fit with threads on the push handle driving lead screw, and an upper end face of the meshing portion is connected to the pressing plate through the compression spring. The pressing plate is fixedly installed on the triangular slider, and the crimping portion is placed below the push rod.

The syringe clamping mechanism includes a clamping plate 10, a spring 26, a baffle plate 27, and a screw 28. The clamping plate includes a clamping plate body, and a telescopic shaft arranged below an inner side of the clamping plate body. A telescopic hole is formed below a middle of the mounting housing. The telescopic shaft of the clamping plate is inserted into the telescopic hole through the spring. The baffle plate abutting against the spring is installed on an end of the telescopic shaft through the screw. A baffle eave for limiting the spring is arranged at the front end of the telescopic hole.

The motor drives the push handle driving lead screw to rotate through the coupling. The push handle driving lead screw is configured to transfer a force to the meshing block 20 on the meshing assembly, and the meshing block 20 drives the triangular slide to linearly move along the guide rods. The triangular slide is connected to the push handle of the syringe through the push rod, and the push handle of the syringe moves as the triangular slide moves, so that the syringe is pushed by the push handle of the syringe for injection of liquid. When the triangular slider moves to a limit position, the optocoupler can detect a signal and send the corresponding signal to the system. When the syringe needs to be replaced, the push handle of the syringe can be rotated counterclockwise to apply a force to the crimping portion of the meshing block 20 through the bump on the push rod, such that the meshing portion is lifted up to be separated from the push handle driving lead screw, so that the push handle of the syringe can freely move along the guide rod in a front and back direction. During the installation of the syringe, the clamping plate can be pulled outwards and rotated clockwise by 90 degrees, and the clamping plate is rotated counterclockwise by 90 degrees and the clamping plate clamps tightly with the syringe under the action of the spring after the syringe is installed. Such structure can be suitable for syringes with different diameters.

A negative pressure energy storage tank in the collection instrument body is a stepless adjustable energy accumulation tank. The stepless adjustable energy accumulation tank includes an energy accumulation tank body 30, an air nozzle 29, a piston 31, a piston driving lead screw 36, a stepping motor 37, a bracket 32, an optocoupler 34, an optocoupler base 33, and an optocoupler baffle 35. The air nozzle is provided on a front end of the energy accumulation tank body, and the energy accumulation tank body is internally with the piston. The piston is driven to expand and retract by the stepping motor arranged at a rear of the energy accumulation tank body through the piston driving lead screw, and the optocoupler baffle is connected to a rear end of the piston driving lead screw. A lower portion of the optocoupler baffle is provided with the optocoupler base, and the optocoupler base is connected to the bracket by a screw. The bracket is installed inside the collection instrument body by screws, and the optocoupler is arranged on a rear end of the optocoupler base.

During operation, the piston driving lead screw rotates with the stepping motor to move forward or backwards to drive the piston to move as the piston driving lead screw is connected to the piston, thus adjusting the volume of the energy accumulation tank. After the volume of the energy storage tank is adjusted to a suitable state, the stepping motor can automatically lock a motor shaft to maintain the volume of the energy accumulation tank. A position where the optocoupler baffle reaches the optocoupler is a zero position.

A central microprocessor controller is arranged inside the collection instrument body. A suction pedal, a negative pressure value setting keyboard, a flushing pedal and a pressure sensor are connected to an input end of the central microprocessor controller. The liquid crystal display on the collection instrument body is bidirectionally connected to the central microprocessor controller. The automatic flushing structure, a negative pressure pump, an automatic pressure-regulating controller, a solenoid valve, the stepless adjustable energy accumulation tank, and an on-off valve are connected to an output end of the central microprocessor controller. The liquid crystal display is a serial screen and a capacitive touch screen in a DC80480F050_3111_0C configuration with a size of 5.0 inch and a resolution of 800*480, which employs a 32-bit processor, supports JPEG pictures, has storage capacity of 128Mbit, employs RS232 communication, and has display accuracy up to ±5 mmHg. The model of the central microprocessor controller is STM32F103C8T6. The model of the negative pressure pump is MB20DC. The model of a pressure sensor is XGZP6857100KPGPN. The model of the solenoid valve is 3V308NCF with a large diameter and fast response. The model of the operating switch is CPV15, and the operating switch is connected to the on-off valve.

The central microprocessor controller controls the negative pressure pump to generate a negative pressure, the generated pressure is regulated through the automatic pressure-regulating controller, and then stored in the stepless adjustable energy accumulation tank. The user controls the solenoid valve to output an expected pressure as needed. The output pressure is constant, and the airflow is stable. The central microprocessor controller controls the automatic flushing structure for injection of liquid, and the automatic flushing structure drives the push handle of the syringe to move through the rotation of the motor, thus injecting the liquid in the syringe fixed to the mounting housing into human body.

In the present disclosure, an automatic flushing structure for flushing in the oocyte retrieval surgery is innovatively designed which has high liquid accuracy, stable flow velocity, capability of saving manpower, reducing the use of pump tube consumables which are indispensable for liquid injection of the peristaltic pump, and avoiding the problem of inconvenient disinfection and sterilization of pump tubes.

The stepless adjustable energy accumulation tank can adjust the capacity thereof in stepless as required. It is easier to establish a target negative pressure when the capacity is small, and an output negative pressure is more stable when the capacity is large.

The system structure composed of the negative pressure pump, the automatic flushing structure, the stepless adjustable energy accumulation tank and the automatic pressure-regulating controller can provide a high-precision negative pressure host system with constant pressure and stable airflow. The whole machine is convenient to operate and use in clinic.

After the collection instrument is turned on, use parameters are set. After the operating switch is pressed down, the stepless adjustable energy accumulation tank is automatically adjusted to the set capacity, and meanwhile, the negative pressure pump is started to make the pressure of the system reach a set value. The pressure of the system is adjusted through the automatic pressure-regulating controller, thus maintaining the negative pressure of the system at the set value. When suction is needed during surgery, the solenoid valve is opened to output a negative pressure by stepping on the suction pedal. When flushing is needed during surgery, by stepping on the flushing pedal, the system can control the automatic flushing structure to inject follicular fluid into the follicle based on to the set fluid volume and flow rate to flush the follicle.

Although the embodiments and accompanying drawings of the present disclosure have been disclosed for purposes of illustration, those skilled in the art may understand that various substitutions, changes and modifications are possible without departing from the spirit and scope of the present disclosure and the appended claims, so the scope of the present disclosure is not limited to the contents disclosed in the embodiments and accompanying drawings.

## Claims

1. A conveniently operable flushing structure with convenient operation, comprising a mounting housing, a syringe driving mechanism, and a syringe clamping mechanism, wherein the mounting housing is of a structure with clamping groove disposed on a side thereof, the syringe clamping mechanism is arranged outside the clamping groove, and the syringe driving mechanism is arranged inside the mounting housing.

2. The conveniently operable automatic flushing structure according to claim 1, wherein the syringe driving mechanism comprises a guide rail frame, a motor, a push handle driving lead screw, a push handle of a syringe, guide rods, a push rod, and a meshing assembly, wherein the push handle driving lead screw driven by the motor is installed inside the guide rail frame, a triangular slider is sleeved on the push handle driving lead screw, and two end corners of the triangular slider are sleeved on the guide rods installed inside the guide rail frame in an up and down direction, and an other end corer of the triangular slider is rotatably connected with the push rod; the push handle of the syringe is connected to an end of the push rod, and the meshing assembly is arranged on a side of the triangular slider; the push rod is configured to control the meshing assembly to be meshed with and separated from the push handle driving lead screw through a bump arranged on the push rod, and a limit optocoupler is installed on one end, away from the motor, of an inner wall of the guide rail frame.

3. The conveniently operable automatic flushing structure according to claim 2, wherein the meshing assembly comprises a meshing block, a compression spring, and a pressing plate; the meshing block comprises a hinged portion, a meshing portion, and a crimping portion; the meshing portion and the crimping portion are arranged on two ends of the hinged portion, respectively; the hinged portion is hinged on the side of the triangular slider; a lower end face of the meshing portion is in fit with threads on the push handle driving lead screw, and an upper end face of the meshing portion is connected to the pressing plate through the compression spring; the pressing plate is fixedly installed on the triangular slider; and the crimping portion is placed below the push rod.

4. The conveniently operable automatic flushing structure according to claim 1, wherein the syringe clamping mechanism comprises a clamping plate, a spring, a baffle plate, and a screw; the clamping plate comprises a clamping plate body, and a telescopic shaft arranged below an inner side of the clamping plate body; a telescopic hole is formed below a middle of the mounting housing; the telescopic shaft of the clamping plate is inserted into the telescopic hole through the spring; and the baffle plate abutting against the spring is installed on an end of the telescopic shaft through the screw.

5. An oocyte collection instrument, comprising a collection instrument body, wherein the automatic flushing structure according to any one of claims 1 to 4 is arranged on a side of the collection instrument body.

6. The oocyte collection instrument according to claim 5, wherein a negative pressure energy storage tank in the collection instrument body is a stepless adjustable energy accumulation tank; the stepless adjustable energy accumulation tank comprises an energy accumulation tank body, an air nozzle, a piston, a piston driving lead screw, a stepping motor, a bracket, an optocoupler, an optocoupler base, and an optocoupler baffle; the air nozzle is provided on a front end of the energy accumulation tank body, and the piston is arranged inside the energy accumulation tank body; the piston is driven to expand and retract by the stepping motor arranged at a rear of the energy accumulation tank body through the piston driving lead screw, and the optocoupler baffle is connected to a rear end of the piston driving lead screw; a lower portion of the optocoupler baffle is provided with the optocoupler base, and the optocoupler base is connected to the bracket by a screw; the bracket is installed inside the collection instrument body by a screw, and the optocoupler is arranged on a rear end of the optocoupler base.

7. The oocyte collection instrument according to claim 5, wherein a central microprocessor controller is arranged inside the collection instrument body, a suction pedal, a negative pressure value setting keyboard, a flushing pedal and a pressure sensor are connected to an input end of the central microprocessor controller; a liquid crystal display on the collection instrument body is bidirectionally connected to the central microprocessor controller; and the automatic flushing structure, a negative pressure pump, an automatic pressure-regulating controller, a venting valve, the stepless adjustable energy accumulation tank, and an on-off valve are connected to an output end of the central microprocessor controller.
